# EUROPEAN PATENT SPECIFICATION

(11) **EP 1 056 461 B1**
(45) Date of publication and mention of the grant of the patent: **18.09.2002**
(21) Application number: 99910932.5
(22) Date of filing: 16.03.1999
(51) Int. Cl.: A61K 31/58, A61P 13/12

(54) **GLUCOCORTICOIDS FOR TREATING GLOMERULONEPHRITIS**
GLUCOCORTICOIDE ZUR BEHANDLUNG VON GLOMERULONEPHITIS
DES GLUCOCORTICOIDES POUR LE TRAITEMENT DE LA GLOMERULONEPHRITE

(30) Priority: 17.03.1998 SE 9800905
(43) Date of publication of application: 06.12.2000
(73) Proprietor: Pharmalink AB, 194 26 Upplands Väsby (SE)
(72) Inventor: HÄLLGREN, Roger, S-740 22 Bälinge (SE); FELLSTRÖM, Bengt, 74 192 Knivsta (SE)
(74) Representative: Bergvall-Eftring, Stina Lena
(86) International application number: SE9900406
(87) International publication number: WO99047144

(56) References cited:
- US-A- 5 643 602
- EUR. CYTOKINE NETW., Volume 5, No. 3, 1994, ECKHARDT J.U. VON ASMUTH et al., "IL-6, IL-8 and TNF Production by Cytokine and Lipopolysaccharide-Stimulated Human Renal Cortical Epithelial Cells In Vitro", pages 301-310.
- STN INTERNATIONAL, File CAPLUS, CAPLUS Accession No. 1993:531139, Dokument No. 119:131139, MINAMI TAKEO et al., "Preliminary Results of Short-Term Combination Immunosuppressions of Mizoribine, Azathioprine and Prednisolone with Pretreatment to Canine Kidney Transplantation"; & J. VET. MED. SCI., (1993), 55(3), 409-14.

## Description

### Field of Invention

The present invention relates to the use of a glucocorticoid selected from budesonide, rofleponide, beclomethasone mono-propionate, beclomethasone di-propionate, ciclesonide, tipredane, flunisolide, triamcinolone acetonide and fluticasone propionate having a first pass metabolism in the fiver of at least 90% which gives a minimal systemic effect, for the manufacture of a medicament for treating glomerulonephritis by oral or rectal administration of a pharmacologically effective amount thereof for release in the intestine.

### Background to the Invention

The functional units of the kidney, such as the glomeruli may suffer from inflammation. An inflammatory attack in the glomeruli is termed glomerulonephritis and can be classified into subgroups such as membraneous glomerulonephritis, focal segmental glomerulosclerosis, mesangial diffuse proliferative glomerulonephritis, endocapillary or extracapillary proliferative glomerulonephritis. Using histopathological techniques these subgroups vary with respect to microscopical or immunohistochemical picture. One cause of inflammation is due to the deposition of immunoglobulin A (IgA) in glomeruli. This condition is termed IgA nephropathy (1-3), and is the most common form of glomerulonephritis in a global perspective.

Assessment of the degree of severity of glomerulonephritis is based on different investigation results. The most important findings are 1) the degree of urinary excretion of protein (proteinuria) and 2) the filtering function of the kidney, which can be assessed by serum creatinine (s-creatinine). Histological examination of material from kidney (renal biopsy) yields information about the type of renal damage as well as the severity of the injury. The outcome of a glomerulonephritis is variable and is dependent upon the histological and the immunohistochemical findings in a renal biopsy. Patients with IgA nephropathy having a constant proteinuria often develop renal failure and uraemia after 5 to 20 years of illness (4).

Various treatments for glomerulonephritis are known. For example substances which act on the immune system, e.g. Cyclophosphamide, Azathioprine and Cyclosporine have been used. Glucocorticoids have also been used (mainly prednisone or prednisone acetate) which may be administered orally or by venous infusion (5, 6). Unfortunately, these treatments cause severe side effects and are not particularly effective. Other suggested treatments include ACE-inhibitors (7), polyunsaturated fatty acid-preparations (8) and vitamin E (9). The treatment results for these therapies for IgA nephropathy have been quite disappointing and it has been concluded that an effective treatment against progressive IgA nephropathy is basically missing (10). For this reason, a substantial number of patients with IgA nephropathy, 20-30%, will eventually develop renal insufficiency and uraemia (1-4). The available treatment for uraemia today is dialysis or kidney transplantation. Renal transplant patients who have been transplanted because of uraemia due to glomerulonephritis frequently suffer from recurrence of glomerulonephritis in the transplant and subsequently a gradual loss of transplant function (11, 12). This is most common with patients who previously suffered from IgA nephropathy. Today there is no effective treatment against recurrence of glomerulonephritis in a transplant.

The glucocorticoids that have been used in IgA nephropathy and in other types of glomerulonephritis are characterised by a substantial gastrointestinal absorption after oral administration, aiming to exert a direct effect on circulating leukocytes and cells that have infiltrated the kidney or the renal transplant, thus having a systemic effect. Such a systemic effect is also achieved if glucocorticoids are administered as an intravenous infusion. Systemic administration of glucocorticoids may have influenced the outcome of IgA nephropathy in some cases.

### Brief description of the invention

Surprisingly, it has now been found that glucocorticoid having a first pass metabolism in the liver of at least 90%, which minimises the systemic effect, is effective in controlling glomerulonephritis and especially IgA nephropathy in a native kidney or a kidney transplant. The substance preferably exerts its effect in the intestinal wall of a certain part of the gut (the lower third of the small intestine and the upper fourth of the large intestine). A man skilled in the art would not have expected that treatment of an apparently healthy intestine should have an effect on an inflamed kidney. This discovery represents a breakthrough in the treatment of glomerulonephritis since it has the advantage of reducing the severe side effects on the body, such as effects on skeleton, metabolism and muscles, caused by therapy with the systemic glucocorticiods used in prior art therapy.

### Summary of the Invention

The invention relates to the use of a glucocorticoid having a first pass metabolism in the liver of at least 90%, which gives a minimal systemic effect, for the manufacturing of a medicament for oral or rectal administration for the treatment of glomerulonephritis. More specifically the invention relates to the use of the glucocorticoid defined above for the manufacturing of a medicament for the treatment of glomeluronephritis, especially IgA nephropathy, in a native kidney or kidney transplant. The medicament is provided in a form by which the active substance is released in a pharmacologically effective amount, in the apparently healthy intestine when it passes the lower third of the small intestine and the upper fourth of the large intestine. The invention relates more specifically to the treatment of IgA nephropathy by administering 0.1mg to 40 mg of the active substance daily to a subject in need thereof.

According to the invention there is further provided a pharmaceutical composition comprising the glucocorticoid, in association with a pharmaceutically acceptable diluent, adjuvant or carrier, which composition is for use in the treatment of glomerulonephritis. For oral use the composition is preferably administered in a form selected from tablets, pills, capsules, syrups, suspensions, powders and granules. The solid forms of the preparation comprise a carrier and an enteric coating, and are most preferably in the form of a capsule comprising microcapsules. When used rectally the active substance is preferably administered in a form selected from foams, suppositories, and enemas.

### Detailed Description of the Invention

The use according to the invention is preferably directed to treating a patient who suffers from acute or chronic glomerulonephritis. Glomerulonephritis may be divided into subtypes such as membranous glomerulonephritis, focal segmental proliferative glomerulonephritis, diffuse mesangioproliferative glomerulonephritis, endocapillary or extracapillary proliferative glomerulonephritis, depending on where the inflammation is located. The use according to this invention is preferably directed to treating the IgA nephropathy type of glomerulonephritis. The invention is particularly suitable for treating patients who suffered from glomerulonephritis (particularly IgA nephropathy), had a transplant, and suffered from a recurrence of glomerulonephritis (particularly IgA nephropathy) in the transplanted kidney.

The glucocorticoid used in the present invention is preferably one which has a first pass metabolism in the liver of at least 90% minimising the systemic effects. The first pass metabolism in the liver of a glucocorticoid substance can be determined using the method disclosed previously (13). More preferably it is budesonide, rofleponide or derivatives thereof, beclomethasone dipropionate, beclomethasone monopropionate, ciclesonide, tipredane, flunisolide, triamcinolone acetonide or flutiscasone propionate. Budesonide, which is a 16,17-butylidenedioxy-11β, 21-dihydroxypregna-1,4-diene-3,20-dione, is particularly preferred.

The glucocorticoid, when administered orally, is generally administered in the form of tablets, pills, capsules, powders or granules, especially in the form of capsules comprising microcapsules. Also liquid preparations such as syrups and suspensions are conceivable. When administered rectally, it is in the form of foams, suppositories or enemas.

The glucocorticoid may be administered as such or as a pharmaceutical composition in combination with a pharmaceutically acceptable diluent, adjuvant or carrier. Particularly preferred are compositions not containing material capable of causing an adverse, e.g. an allergic reaction.

The glucocorticoid substance may be admixed with an adjuvant or a carrier, e.g. lactose, saccharose, sorbitol, mannitol; starches such as potato starch, corn starch or amylopectin; cellulose derivatives; a binder such as gelatin or polyvinlypyrrolidone; and a lubricant such as magnesium stearate, calcium stearate, polyethylene glycol, waxes and/or paraffin, and then compressed into tablets. If coated tablets are required, the cores, prepared as described above, may be coated with a concentrated sugar solution, which may contain e.g. gum arabic, gelatin, talcum and/or titanium dioxide. Alternatively, the tablet may be coated with a suitable polymer dissolved in a readily volatile organic solvent. The tablet preferably has an enteric coating to allow release of the glucocorticoid substance in the lower intestine. Suitable capsules may be prepared by using the methods described in EP-A-502092, WO 97/27843 or WO 95/08323.

For the preparation of soft gelatin capsules, the glucocorticoid substance may be admixed with e.g. a vegetable oil or polyethylene glycol. Hard gelatin capsules may contain granules of the substance using either the above mentioned excipients for tablets, e.g. lactose, saccharose, sorbitol, mannitol, starches, cellulose derivatives or gelatin. Also liquid or semisolid formulations of the glucocorticoid substance may be filled into hard gelatin capsules.

Liquid preparations of oral application may be in the form of syrups or suspensions, for example solutions containing the glucocorticoid substance, the balance being sugar and a mixture of ethanol, water, glycerol and propylene glycol. Optionally such liquid preparations may contain colouring agents, flavouring agents, saccharine and carboxymethylcellulose as a thickening agent or other excipients known to those skilled in the art.

Rectal enema formulations can be in the form of simple suspensions of the glucocorticoid substance in a pharmaceutically acceptable carrier or may be in the form of a rectal foam formulation, for example as described in EP-A-468555.

The glucocorticoid substance is preferably administered at a dosage regime from 0.1 to 40 mg, more preferably from 0.5 to 20 mg, most preferably from 1 to 10 mg, either as a single dose or in divided doses from 2 to 4 times per day. The pharmaceutical composition for oral administration used in the present invention should preferably be prepared in such a way that the glucocorticoid substance is released during the passage of the lower third of the small intestine and the upper fourth of the large intestine. This is in order to achieve a high local concentration of glucocorticoid in these parts of the intestine so that the glucocorticoid exerts its effect, preferably through the intestinal wall of these parts of the intestine.

The invention is illustrated by the following examples where budesonide was administered orally using the Entocort™ preparation (tablet form) to patients suffering from IgA nephropathy in native kidneys or kidney transplants.

### Example 1

A 52-year old man fell ill with signs of renal disorder as indicated by proteinuria and red blood cells in the urine in 1982. After renal biopsy and histological analysis of renal tissue he was diagnosed with IgA nephropathy. He was treated with various antihypertensive drugs but the proteinuria increased and by 1990 he had developed renal insufficiency and later that year he developed uraemia which in turn necessitated dialysis treatment. In 1993 he received a kidney from a deceased person. The transplanted kidney was working satisfactorily for the first 24 months and the patient was treated with glucocorticoid substance with systemic effect (prednisolone) as well as with an immunosuppressive drug (Cyclosporine). In 1995 the first signs of renal disorder of the transplanted kidney were detected with increased proteinuria and reduced renal function as measured by changes in serum-creatinine concentrations. After renal biopsy of the transplant followed by histological analysis it was shown that the tissue was affected by IgA nephropathy. At this stage, treatment with budesonide (Entocort™, 9mg/day) was initiated. Before the treatment commenced he had a considerable proteinuria (3.1 g albumin/day; normal range 0.3 g/day) and a reduced renal filtering capacity (serum creatinine 264 µmol/l; normal range 80-115 µmol/l). After treatment with budesonide both the proteinuria and the renal function improved significantly as shown in Table 1.

**Table 1**

| Time (weeks) | U-albumin (mg/24h) | S-creatinine (µmol/l) |
|---|---|---|
| 0 | 3089 | 264 |
| 6 | 624 | 213 |
| 12 | 347 | 203 |

### Example 2

A 29-year old patient (female) with IgA nephropathy, where histological examination of material from a renal biopsy disclosed irregular widening of the mesangium and a slight increase of mesangial matrix, but no cellular proliferation. She had earlier been treated with immunosuppressive and antihypertensive drugs without any success as regards improvement of renal function or decrease of proteinuria. Treatment with budesonide (9 mg/day) was initiated and after three months of treatment a 50% reduction of proteinuria was detected as disclosed in Table 2.

**Table 2**

| Time (weeks) | U-albumin (mg/24h) | S-creatinine (µmol/l) |
|---|---|---|
| 0 | 899 | 85 |
| 6 | 745 | 75 |
| 12 | 421 | 75 |

### Example 3

A 47-year old patient (male) suffered from IgA nephropathy, which was diagnosed in August 1996. Histological examination of material from renal biopsy showed a slight to moderate widening of the mesangium, slight increase of mesangial matrix and a slight mesangial proliferation. Furthermore, focal chronic inflammation was present in the interstitium and there was focal fibrosis and partial atrophic tubuli present. Treatment with budesonide (9 mg/day) was initiated and after 12 weeks of treatment the proteinuria was reduced and the renal function (serum creatinine) was improved as shown in Table 3.

**Table 3**

| Time (weeks) | U-albumin (mg/24h) | S-creatinine (µmol/l) |
|---|---|---|
| 0 | 1349 | 147 |
| 6 | 1050 | 142 |
| 12 | 1067 | 129 |

### Example 4

A patient, 37-years old, (male) with IgA nephropathy, where histological examination of the renal biopsy demonstrated that 2/15 glomeruli were sclerotic and the other glomeruli had mesangial proliferative changes. A slight focal interstitial fibrosis and tubular atrophy could also be demonstrated. Treatment with budesonide (9 mg/day) was initiated and after 12 weeks of treatment the proteinuria was reduced and renal function was basically unchanged as shown in Table 4.

**Table 4**

| Time (weeks) | U-albumin (mg/24h) | S-creatinine (µmol/l) |
|---|---|---|
| 0 | 1244 | 116 |
| 6 | 964 | 113 |
| 12 | 1078 | 112 |

### Example 5

A 52-year old patient (female) with IgA nephropathy, where the histological examination of material from renal biopsy showed substantial segmental sclerotic changes in 2-4/15 glomeruli and slight mesangial proliferative changes in the rest of the glomeruli. There was also slight interstitial fibrosis and tubular atrophy present. Treatment with budesonide (9 mg/day) was initiated and after 12 weeks of treatment the proteinuria was reduced and the renal function was possibly improved as shown in Table 5.

**Table 5**

| Time (weeks) | U-albumin (mg/24h) | S-creatinine (µmol/l) |
|---|---|---|
| 0 | 634 | 106 |
| 6 | 516 | 107 |
| 12 | 431 | 100 |

### Example 6

A 26-year old patient (male) with IgA nephropathy was studied before and during the treatment with budesonide (9 mg/day). After 12 weeks of treatment the proteinuria was reduced as shown in Table 6.

**Table 6**

| Time (weeks) | U-albumin (mg/24h) | S-creatinine (µmol/l) |
|---|---|---|
| 0 | 1449 | 91 |
| 6 | 1398 | 97 |
| 12 | 1100 | 90 |

### Example 7

A 27-year old patient (female) with IgA nephropathy, where histological examination of material from the renal biopsy showed an irregular widening of the mesangium and a focal increase of mesangial matrix and a slight mesangial proliferation. The interstitium, tubuli, and vessels had normal appearances. Treatment with budesonide (9 mg/day) was initiated and after 12 weeks of treatment the proteinuria was reduced as shown in Table 7.

**Table 7**

| Time (weeks) | U-albumin (mg/24h) | S-creatinine (µmol/l) |
|---|---|---|
| 0 | 311 | 82 |
| 6 | 212 | 81 |
| 12 | 167 | 81 |

### Example 8

A 36-year old patient (male) with IgA nephropathy, where histological examination of material from renal biopsy showed that 14/28 glomeruli were entirely sclerotic and in the rest of glomeruli there was a widening of the mesangium and a slight mesangial proliferation and an increase of mesangial matrix. Focal fibrosis was also found in the interstitium. Treatment with budesonide (9 mg/day) was initiated and after 6 weeks of treatment the proteinuria was reduced and renal function was improved as shown in Table 8.

**Table 8**

| Time (weeks) | U-albumin (mg/24h) | S-creatinine (µmol/l) |
|---|---|---|
| 0 | 829 | 171 |
| 6 | 596 | 152 |

### References

1. Clarkson et al. In Diseases of the Kidney, Braun & Co, 1988, pp 2061-2090.
2. Alarmaatine et al. Clin Nephrol 34 (2): 45, 1990.
3. Lai et al. Int J Artif Organs 17 (9): 457, 1994.
4. Scheinman et al. Nephron 75: 251, 1997.
5. Shu et al. Clin Nephrol 44: 86, 1995.
6. Goomanos et al. NDT 10: 1173, 1995.
7. Schmidt et al. Curr Op Nephrol Hypertension 5: 552, 1996.
8. Donadio et al. NEJM 3: 1194, 1994.
9. Trachtman et al. Ped Res 40: 620, 1996.
10.Feehally et al. Curr Op Nephrol Hypertension 5: 442, 1996.
11.Frohnert et al. Clin Transpl 11: 127, 1997.
12.Odum et al. NDT 9: 309, 1994.
13.Andersson P et al. Xenobiotica 17: 5, 1987.

## Claims

1. Use of a glucocorticoid selected from budesonide, rofleponide, beclomethasone mono-propionate, beclomethasone di-propionate, ciclesonide, tipredane, flunisolide, triamcinolone acetonide and fluticasone propionate having a first pass metabolism in the fiver of at least 90% which gives a minimal systemic effect, for the manufacture of a medicament for treating glomerulonephritis by oral or rectal administration of a pharmacologically effective amount thereof for release in the intestine.

2. The use according to claim 1, wherein the glucocorticoid is budesonide.

3. The use according to any of claims 1 to 2 wherein the daily pharmacologically effective amount is from 0.1 mg to 40 mg, preferably 0.5 mmg to 20 mg, more preferably 1mg to 10 mg, administered as a single dose or in divided doses 2 to 4 times daily.

4. The use according to any of claims 1-3, wherein the medicament is designed for oral administration.

5. The use according to claim 4 of the medicament in a form selected from tablets, pills, capsules, powders, syrups, solutions and granules, and wherein the solid forms comprise a carrier and an enteric coating.

6. The use according to claim 5, wherein the medicament is in the form of a capsule comprising microcapsules.

7. The use according to claims 4 to 6, wherein said pharmacologically effective amount of glucocorticoid is released when passing the lower third of the small intestine and the upper fourth of the large intestine.

8. The use according to any of claims 1-3, wherein the medicament is designed for rectal administration.

9. The use according to claim 8, wherein the medicament is selected from enemas, suppositories and foams.

10. The use according to any of claims 1 to 9 of the medicament for the treatment of IgA nephropathy in a native kidney or kidney transplant.

## Patentansprüche

1. Verwendung eines Glucocorticoids, ausgewählt aus Budesonid, Rofleponid, Beclomethasonmonopropionat, Beclomethasondipropionat, Ciclesonid, Tipredan, Flunisolid, Triamcinolonacetonid und Fluticasonpropionat, mit einem first pass-Metabolismus in der Leber von mindestens 90%, das eine minimale systemische Wirkung bereitstellt, zur Herstellung eines Medikaments für die Behandlung von Glomerulonephritis durch orale oder rektale Verabreichung einer pharmakologisch wirksamen Menge davon zur Freisetzung von Intestin.

2. Verwendung gemäß Anspruch 1, wobei das Glucocorticoid Budesonid ist.

3. Verwendung gemäß einem der Ansprüche 1 oder 2, wobei die tägliche, pharmakologisch wirksame Menge 0,1 mg bis 40 mg, vorzugsweise 0,5 mg bis 20 mg, stärker bevorzugt 1 mg bis 10 mg, verabreicht als Einzeldosis oder in aufgeteilten Dosen 2 bis 4 mal täglich, beträgt.

4. Verwendung gemäß einem der Ansprüche 1 bis 3, wobei das Medikament zur oralen Verabreichung bestimmt ist.

5. Verwendung gemäß Anspruch 4 des Medikaments in einer aus Tabletten, Pillen, Kapseln, Pulvern, Sirupen, Lösungen und Granulaten ausgewählten Form, wobei die festen Formen einen Träger und eine essbare Beschichtung umfassen.

6. Verwendung gemäß Anspruch 5, wobei das Medikament in Form einer Mikrokapseln umfassenden Kapsel vorliegt.

7. Verwendung gemäß den Ansprüchen 4 bis 6, wobei die pharmakologisch wirksame Menge von Glucocorticoid beim Durchlauf des unteren Drittels des kleinen Intestins und des oberen Viertels des großen Intestins freigesetzt wird.

8. Verwendung gemäß einem der Ansprüche 1 bis 3, wobei das Medikament zur rektalen Verabreichung bestimmt ist.

9. Verwendung gemäß Anspruch 8, wobei das Medikament aus Klistieren, Zäpfchen und Schäumen ausgewählt ist.

10. Verwendung gemäß einem der Ansprüche 1 bis 9 des Medikaments zur Behandlung von IgA-Nierenschädigung in natürlicher Niere oder Nierentransplantat.

## Revendications

1. Utilisation d'un glucocorticoïde choisi parmi le budésonide, le rofléponide, le béclométasone monopropionate, le béclométasone dipropionate, le ciclésonide, le tiprédane, le flunisolide, le triamcinolone acétonide et le fluticasone propionate, ayant un métabolisme de premier passage hépatique d'au moins 90 % qui produit un effet systémique minimal, pour la fabrication d'un médicament destiné à traiter la glomérulonéphrite par administration par voie orale ou rectale d'une quantité pharmacologiquement efficace de celui-ci pour une libération dans l'intestin.

2. Utilisation selon la revendication 1, dans laquelle le glucocorticoïde est le budésonide.

3. Utilisation selon l'une quelconque des revendications 1 à 2, dans laquelle la quantité pharmacologiquement efficace quotidienne est comprise entre 0,1 mg et 40 mg, de préférence entre 0,5 mg et 20 mg, de manière plus préférentielle entre 1 mg et 10 mg, administrée en une dose unique ou en doses fractionnées 2 à 4 fois par jour.

4. Utilisation selon l'une quelconque des revendications 1 à 3, dans laquelle le médicament est conçu pour l'administration par voie orale.

5. Utilisation selon la revendication 4 du médicament sous une forme choisie parmi les comprimés, les pilules, les capsules, les poudres, les sirops, les solutions et les granulés, et dans laquelle les formes solides comprennent un véhicule et un enrobage entérique.

6. Utilisation selon la revendication 5, dans laquelle le médicament se présente sous la forme d'une capsule comprenant des microcapsules.

7. Utilisation selon les revendications 4 à 6, dans laquelle ladite quantité pharmacologiquement efficace de glucocorticoïde est libérée lorsqu'elle passe le tiers inférieur de l'intestin grêle et le quart supérieur du gros intestin.

8. Utilisation selon l'une quelconque des revendications 1 à 3, dans laquelle le médicament est conçu pour l'administration rectale.

9. Utilisation selon la revendication 8, dans laquelle le médicament est choisi parmi les lavements, les suppositoires et les mousses.

10. Utilisation selon l'une quelconque des revendications 1 à 9 du médicament pour le traitement de la néphropathie à IgA dans un rein natif ou un transplant rénal.
